# EUROPÄISCHE PATENTSCHRIFT

(11) **EP 0 648 754 B1**
(45) Veröffentlichungstag und Bekanntmachung des Hinweises auf die Patenterteilung: **12.07.2000**
(21) Anmeldenummer: 94810587.9
(22) Anmeldetag: 06.10.1994
(51) Int. Cl.: C07D 251/24, C07D 251/10, C08K 5/3492

(54) **Verfahren zur Herstellung von Hydroxyphenyl-1,3,5-Triazinen**
Process for the preparation of hydroxyphenyl-1,3,5-triazines
Procédé pour la préparation d'hydroxyphényl-1,3,5-triazines

(30) Priorität: 15.10.1993 CH 311693
(43) Veröffentlichungstag der Anmeldung: 19.04.1995
(73) Patentinhaber: Ciba Specialty Chemicals Holding Inc., 4057 Basel (CH)
(72) Erfinder: Reinehr, Dieter, Dr., D-79400 Kandern (DE); Bacher, Jean Pierre, D-68220 Buschwiller (FR)

(56) Entgegenhaltungen:
- EP-A- 0 444 323
- EP-A- 0 497 734
- EP-A- 0 604 980
- WO-A-94/05645
- US-A- 3 118 887
- US-A- 3 268 474
- US-A- 5 545 836
- Khim.Geteritsikl.Soedin., 2(1969), p.350-353
- A.R. KATRITZKY ET.AL.: "", COMPREHENSIVE HETEROCYCLIC CHEMISTRY, Oxford, 1984, Band 3, Nr. 2B, Seiten 492 - 517

## Beschreibung

Die vorliegende Erfindung betrifft ein Verfahren zur Herstellung von hydroxyphenylsubstituierten 1,3,5-Triazinen, hydroxyphenylsubstituierte Dihydrotriazinverbindungen, ein Verfahren zur Herstellung dieser Verbindungen, die Verwendung der Dihydrotriazinverbindungen als Ausgangsverbindungen zur Herstellung von UV-Absorbern sowie der 1,3,5-Triazinverbindungen als UV-Absorber.

Die Oxidation von Dihydro-Triazinverbindungen zu 1,3,5-Triazinen mit Hilfe von Chloranil und guten Ausbeuten ist aus der Khim. Geteritsikl. Soedin. (2), S. 350-353 (1969) bekannt. Mit anderen Oxidationsmitteln sind keine einheitlichen Produkte herstellbar. Das Oxidationsmittel Chloranil ist allerdings aus ökologischer Sicht sehr bedenklich. Weitere Synthesen von Triazinen sind beschrieben in US-A-3118887, US-A-3268474, EP-A-444323, A.R. Katritzky et al. (Ed.), Comprehensive Heterocyclic Chemistry, vol. 3(2B), p.492-517 (1984), wobei letztere Publikation auch einzelne Methoden zur Herstellung von Dihydrotriazinen nennt.

Aufgabe der vorliegenden Erfindung ist es, 1,3,5-Triazine ausgehend von Dihydrotriazinen auf anderem Wege herzustellen.

Überraschenderweise wurde nun gefunden, dass die Oxidation von Dihydrotriazinverbindungen zu den entsprechenden 1,3,5-Triazinen mit bestimmten Verbindungen, die gewöhnlich als Reduktionsmittel verwendet werden, auf einfache Weise und in guten Ausbeuten gelingt. Gegenstand der vorliegenden Erfindung ist daher ein Verfahren zur Herstellung einer Triazinverbindung der Formel 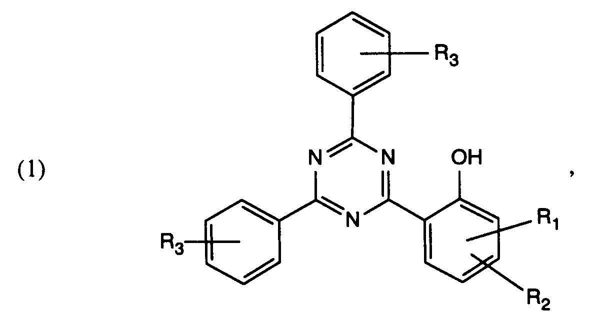 das dadurch gekennzeichnet ist, dass man eine Dihydrotriazinverbindung der Formel 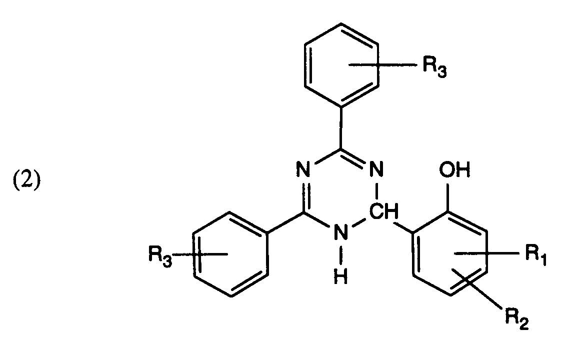 mit einem Reduktionsmittel vom Typ Dithionit, Pyrosulfit, Sulfit oder Thiosulfit in die Verbindung der Formel (1) überführt,
wobei in den Formeln (1) und (2)
R₁, R₂ und R₃ unabhängig voneinander Wasserstoff, Halogen, C₁-C₁₈-Alkyl,
C₁-C₁₈-Alkoxy oder -C≡N bedeuten.

C₁-C₁₈-Alkyl und C₁-C₁₈-Alkoxy sind geradkettige oder verzweigte Alkyl- bzw. Alkoxyreste wie z.B. Methyl, Ethyl, n-Propyl, Isopropyl, n-Butyl, sek.Butyl, tert.Butyl, Amyl, Isoamyl oder tert.Amyl, Heptyl, Octyl, Isooctyl, Nonyl, Undecyl, Dodecyl, Tetradecyl, Pentadecyl, Hexadecyl, Heptadecyl oder Octadecyl bzw. Methoxy, Ethoxy, Propoxy, Butoxy, Pentyloxy, Hexyloxy, Heptyloxy, Octyloxy, Isooctyloxy, Nonyloxy, Undecyloxy, Dodecyloxy, Tetradecyloxy, Pentadecyloxy, Hexadecyloxy, Heptadecyloxy oder Octadecyloxy.

Halogen bedeutet Chlor, Brom oder Jod. Chlor ist bevorzugt.

Als Reduktionsmittel für das vorliegende Verfahren werden vorzugsweise Natriumbisulfit oder Natriumdithionit eingesetzt.

Die Reaktion kann bei einer Temperatur von 0 bis 150°C, vorzugsweise von 20 bis 80°C durchgeführt werden. Die Reaktionszeit beträgt in der Regel 1 bis 20, vorzugsweise 2 bis 8 Stunden.

Die Reaktion wird gewöhnlich in einem polaren Lösungsmittel durchgeführt. Als Lösungsmittel kommen beispielsweise in Betracht: Dimethylformamid (DMF), Dimethylacetamid, Dimethylsulfoxid, N-Methylpyrrolidon oder Sulfolan. Vorzugsweise werden als Lösungsmittel DMF oder ein Gemisch aus Wasser und DMF, Dimethylacetamid (DMA) oder ein Gemisch aus Dimethylacetamid und Wasser verwendet.

Für das erfindungsgemässe Herstellungsverfahren kommen vorzugsweise Dihydrotriazine der Formel (1) in Betracht, bei denen
R₁ Wasserstoff, 4-OCH₃ oder 3-OCH₃ und
R₂ und R₃ unabhängig voneinander Wasserstoff oder 4-OCH₃ bedeuten.

Die Dihydrotriazine der Formel (1) sind zum Teil aus der Khim. Geteritsikl. Soedin. (2), S. 350-353 (1969) bekannt. Zum Teil stellen sie auch neue Verbindungen dar. Sie entsprechen der Formel 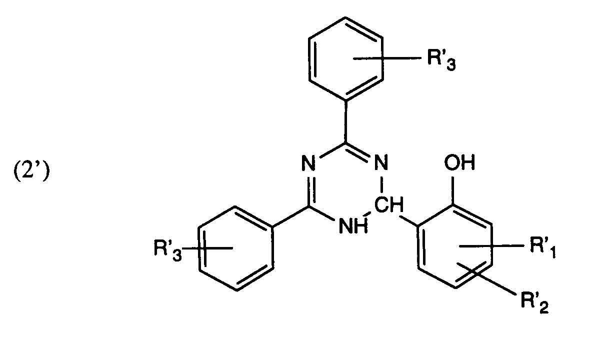 worin
R'₁, R'₂ und R'₃ unabhängig voneinander Wasserstoff, Halogen, C₁-C₁₈-Alkyl,
C₁-C₁₈-Alkoxy oder -C≡N bedeuten, mit der Massgabe, dass R'₁, R'₂ und R'₃ nicht gleichzeitig Wasserstoff bedeuten.

Die Herstellung dieser neuen Dihydrotriazine erfolgt in an sich bekannter Weise durch Umsetzung von 2 Mol eines Benzamidinhydrohalogenids der Formel 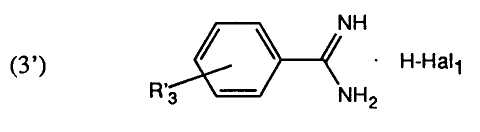 mit einem Mol eines
α-Hydroxybenzaldehyds der Formel

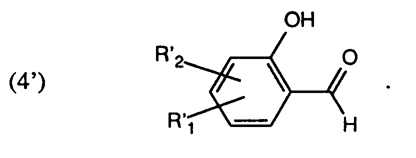

R'₁ und R'₂ haben die in Formel (2') angegebene Bedeutung. Hal₁ bedeutet Halogen.

Als Benzamidinhydrohalogenid kommen das Benzamidinhydrobromid und vorzugsweise das -chlorid in Betracht.

Die Dihydrotriazinverbindungen der Formeln (2) und (2') stellen Ausgangsprodukte zur Herstellung von UV-Absorbern dar. Die Triazinverbindungen der Formel (1) stellen wertvolle UV-Absorber für organische Materialien, insbesondere Polyesterfasermaterialien dar und zeichnen sich durch gute Eigenschaften aus.

Die folgenden Beispiele veranschaulichen die Erfindung. Prozentangaben beziehen sich auf das Gewicht.

### Herstellungsbeispiele

### Beispiel 1:

103 g (0,6 Mol) Benzamidinhydrochlorid (ca. 91%ig) werden zusammen mit 108 g (0,6 Mol) einer 30%igen methanolischen Natriummethylatlösung in 100 ml Methanol vorgelegt. Es entsteht eine weiße Suspension, in die 65,1 g (0,3 Mol) 2-Hydroxy-4-methoxy-benzaldehyd (69,4 %ige Lösung in DMF) eingetropft werden, wobei die Farbe nach ockergelb umschlägt. Es wird auf 38-40°C erwärmt und bei dieser Temperatur während 16 Stunden gerührt. Sodann wird auf 10°C abgekühlt, abfiltriert und mit 300 ml Methanol/Wasser im Verhältnis 1:1 und dann mit 1,5 l Wasser gewaschen. Nach dem Trocknen im Vakuum bei 110°C erhält man ein fast farbloses Produkt der Formel 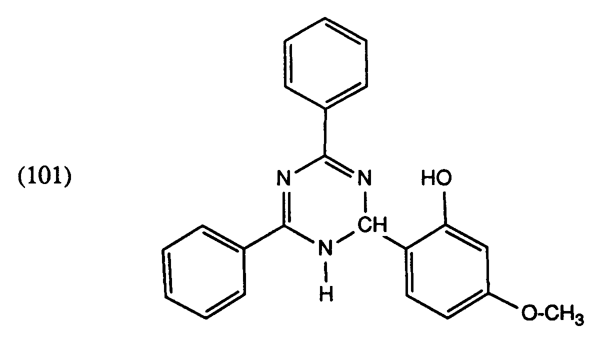
Ausbeute: 72 g (67,2 % d. Th.)
Fp.: 173-174°C

### Beispiel 2:

35,7 g (0,1 Mol) des in Beispiel 1 hergestellten Produktes werden in 350 ml Dimethylformamid (DMF) gelöst. Bei 45°C werden 38,3 ml einer 40%igen wässrigen Natriumbisulfitlösung innerhalb von 30 Minuten zugetropft. Es wird 6 Stunden bei 48°C gerührt, danach mit 300 ml Wasser versetzt, abgekühlt und filtriert. Nach dem Trocknen im Vakuum bei 110°C erhält man das hellbeige gefärbte Produkt der Formel 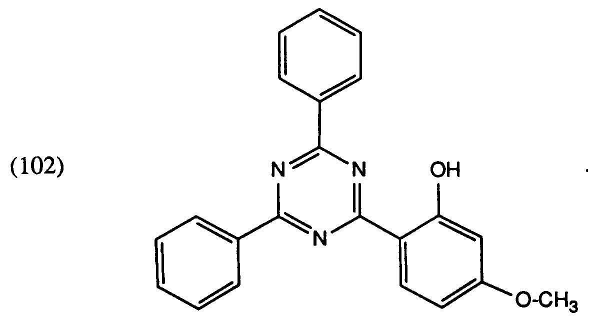
Ausbeute: 32,5 g (91,5% d. Th.)
Fp.: 203°C

### Beispiel 3:

Man verfährt wie in Beispiel 2 beschrieben mit dem Unterschied, dass man 350 ml Dimethylacetamid anstelle von 350 ml DMF verwendet. Man erhält die Verbindung der Formel (102) mit einer Ausbeute von 89% d.Th.

### Beispiel 4:

Man verfährt wie in Beispiel 1 beschrieben, mit dem Unterschied, dass man 0,3 Mol Salicylaldehyd anstelle von 0,3 Mol 2-Hydroxy-4-methoxy-benzaldehyd verwendet. Man erhält das Dihydrotriazinprodukt der Formel 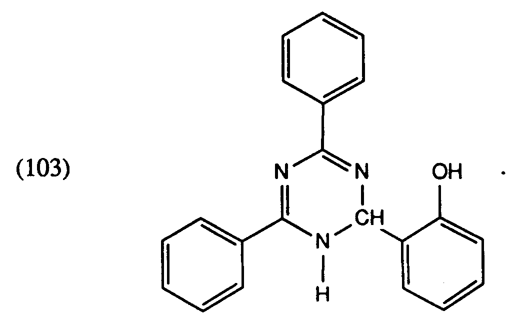
Ausbeute: 94 g (96 % d. Th)
Fp.: 182°C

### Beispiel 5:

Man verfährt wie in Beispiel 2 beschrieben, jedoch unter Verwendung von 32,3 g (0,098 Mol) des in Beispiel 4 hergestellten Produktes. Man erhält das Reaktionsprodukt der Formel 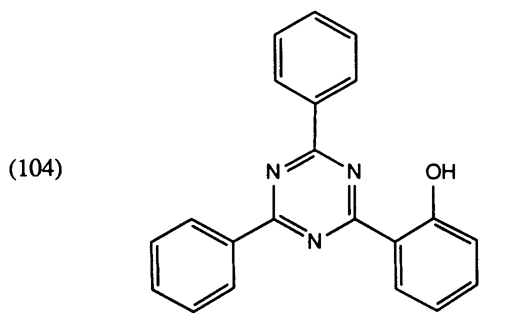 als leicht gelbliches Produkt.
Ausbeute: 22 g (69 % d. Th.)
Fp.: 246-247°C

### Beispiel 6:

Verwendet man in Beispiel 5 anstelle von Natriumbisulfit Natriumdithionit, so erhält man das Reaktionsprodukt der Formel (104) in einer Ausbeute von 65% d. Th.

### Beispiele 7 bis 9:

Analog Beispiel 1 lassen sich die folgenden Dihydrotriazine herstellen (Tabelle 1):

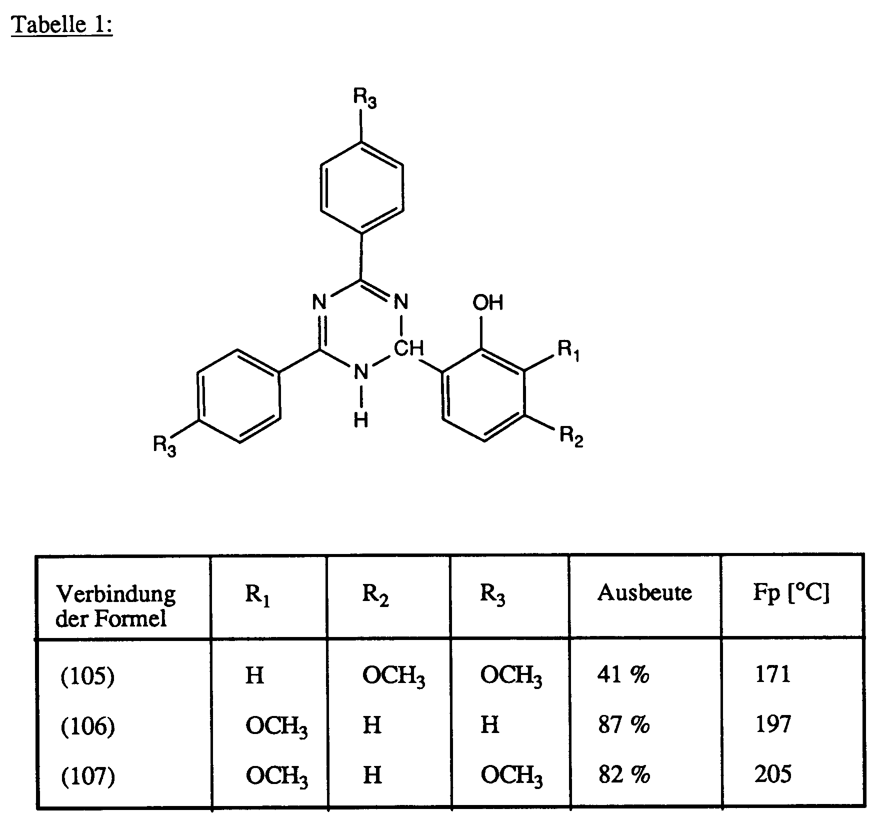

### Beispiele 10 bis 12:

Die in den Beispielen 7 bis 9 hergestellten Dihydrotriazine werden analog Beispiel 2 in die entsprechenden Triazine überführt (Tabelle 2).

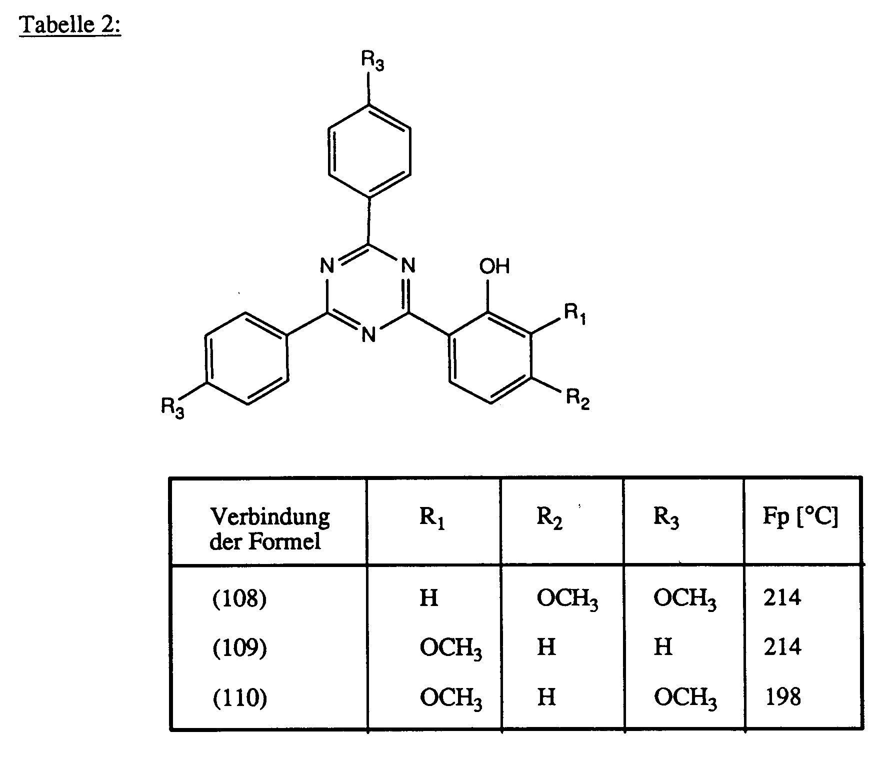

## Patentansprüche

1. Verfahren zur Herstellung einer Triazinverbindung der Formel 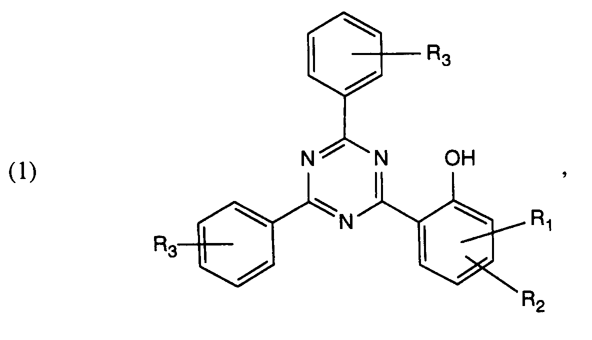 dadurch gekennzeichnet, dass man eine Dihydrotriazinverbindung der Formel 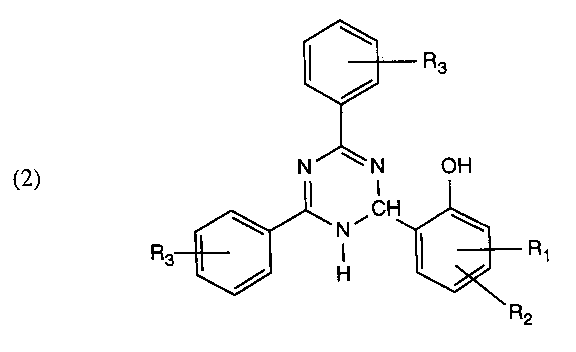 mit einem Reduktionsmittel vom Typ Dithionit, Pyrosulfit, Sulfit oder Thiosulfit in die Verbindung der Formel (1) überführt,
wobei in den Formeln (1) und (2)
R₁, R₂ und R₃ unabhängig voneinander Wasserstoff, Halogen, C₁-C₁₈-Alkyl,
C₁-C₁₈-Alkoxy oder -C≡N bedeuten.

2. Verfahren nach Anspruch 1, dadurch gekennzeichnet, dass man als Reduktionsmittel Natriumdithionit oder Natriumbisulfit verwendet.

3. Verfahren nach Anspruch 1 oder 2, dadurch gekennzeichnet, dass man die Reaktion bei einer Temperatur von 0 bis 150°C durchführt.

4. Verfahren nach einem der Ansprüche 1 bis 3, dadurch gekennzeichnet, dass die Reaktion in einem polaren Lösungsmittel oder Lösungsmittelgemisch durchgeführt wird.

5. Verfahren nach Anspruch 4, dadurch gekennzeichnet, dass man als Lösungsmittel Dimethylformamid, Dimethylacetamid, Dimethylsulfoxid, N-Methylpyrrolidon oder Sulfolan verwendet.

6. Verfahren nach Anspruch 4 oder 5, dadurch gekennzeichnet, dass das Lösungsmittel als Gemisch mit Wasser vorliegt.

7. Verfahren nach einem der Ansprüche 1 bis 6, dadurch gekennzeichnet, dass
R₁ Wasserstoff, 4-OCH₃ oder 3-OCH₃,
R₂ und R₃ unabhängig voneinander Wasserstoff oder 4-OCH₃ bedeuten.

8. Verwendung einer Dihydrotriazinverbindung der Formel (2') 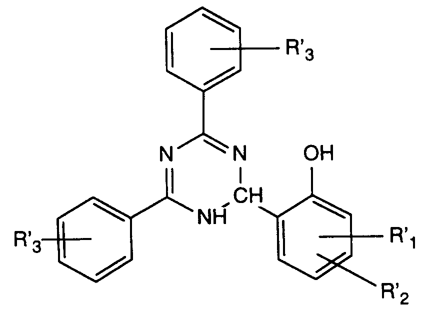 worin
R'₁, R'₂ und R'₃ unabhängig voneinander Wasserstoff, C₁-C₁₈-Alkyl, C₁-C₁₈-Alkoxy, Halogen oder -C≡N
bedeuten, mit der Massgabe dass R'₁, R'₂ und R'₃ nicht gleichzeitig Wasserstoff bedeuten, als Zwischenprodukt für die Herstellung von UV-Absorbern der Formel (1) gemäß Anspruch 1.

## Claims

1. A process for the preparation of a triazine compound of formula 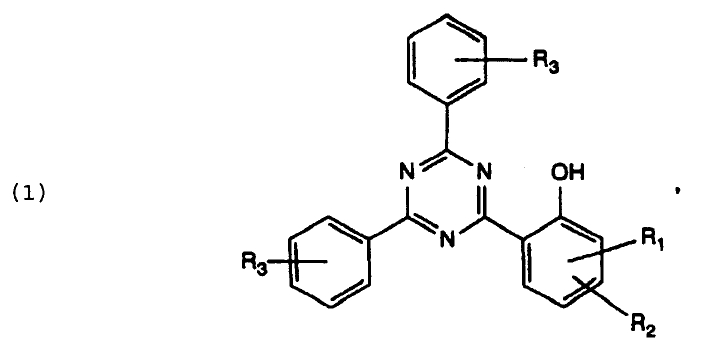 which comprises converting a dihydrotriazine compound of formula 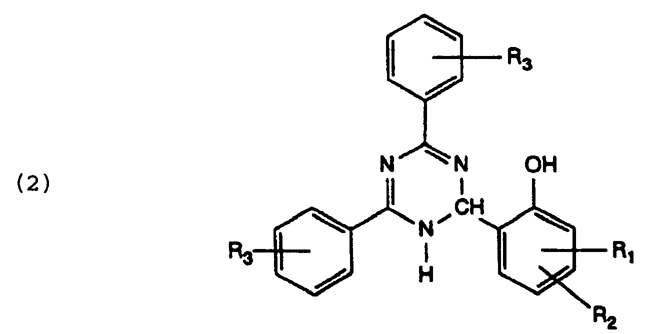 with a dithionite, pyrosulfite, sulfite or thiosulfite type reducing agent to the compound of formula (1), where in formulae (1) and (2)
R₁, R₂ and R₃ are each independently of one another hydrogen, halogen, C₁-C₁₈alkyl, C₁-C₁₈alkoxy or -C≡N.

2. A process according to claim 1, wherein the reducing agent is sodium dithionite or sodium bisulfite.

3. A process according to claim 1 or 2, wherein the reaction is carried out at a temperature from 0 to 150°C.

4. A process according to any one of claims 1 to 3, wherein the reaction is carried out in a polar solvent or solvent mixture.

5. A process according to claim 4, wherein the solvent used is dimethylformamide, dimethylacetamide, dimethyl sulfoxide, N-methylpyrrolidone or sulfolane.

6. A process according to claim 4 or 5, wherein the solvent is a mixture with water.

7. A process according to any one of claims 1 to 6, wherein
R₁ is hydrogen, 4-OCH₃ or 3-OCH₃,
R₂ and R₃ are each independently of the other hydrogen or 4-OCH₃.

8. The use of a dihydrotriazine compound of formula (2') 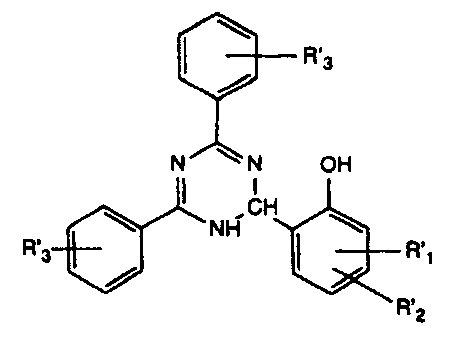 wherein
R'₁, R'₂ and R'₃ are each independently of one another hydrogen, C₁-C₁₈alkyl, C₁-C₁₈alkoxy, halogen or -C≡N, with the proviso that R'₁, R'₂ or R'₃ are not simultaneously hydrogen, as an intermediate for preparing UV absorbers of the formula (1) as set forth in claim 1.

## Revendications

1. Procédé de préparation d'un composé de triazine de formule 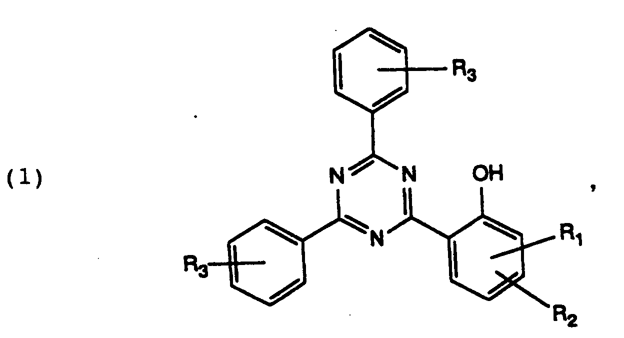 caractérisé en ce qu'on transforme un composé de dihydrotriazine de formule 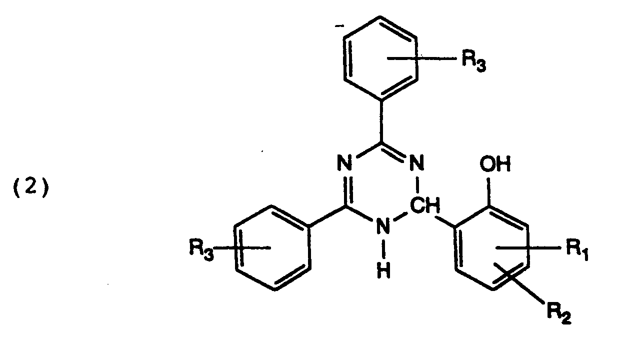 à l'aide d'un réducteur du type dithionite, pyrosulfite, sulfite ou thiosulfite en les composés de formule (1), dans les formules (1) et (2)
R₁, R₂ et R₃ représentant, indépendamment l'un de l'autre, des atomes d'hydrogène, des atomes d'halogène, des groupes alkyle en C₁-C₁₈, alkoxy en C₁-C₁₈ ou C≡N.

2. Procédé selon la revendication 1, caractérisé en ce qu'on utilise comme réducteur le dithionite de sodium ou le bisulfite de sodium.

3. Procédé selon la revendication 1 ou 2, caractérisé en ce qu'on met en oeuvre la réaction à une température de 0 à 150°C.

4. Procédé selon l'une des revendications 1 à 3, caractérisé en ce qu'on met en oeuvre la réaction dans un solvant ou mélange de solvants polaires.

5. Procédé selon la revendication 4, caractérisé en ce qu'on emploie comme solvant le diméthylformamide, le diméthylacétamide, le diméthylsulfoxyde, la N-méthylpyrrolidone ou la sulfolane.

6. Procédé selon la revendication 4 ou 5, caractérisé en ce que le solvant se présente sous forme d'un mélange avec l'eau.

7. Procédé selon l'une des revendications 1 à 6, caractérisé en ce que
R₁ représente un atome d'hydrogène, des groupes 4-OCH₃ ou 3-OCH₃,
R₂ et R₃ représentent, indépendamment l'un de l'autre, des atomes d'hydrogène ou un groupe 4-OCH₃.

8. Utilisation d'un composé de dihydrotriazine de formule (2') 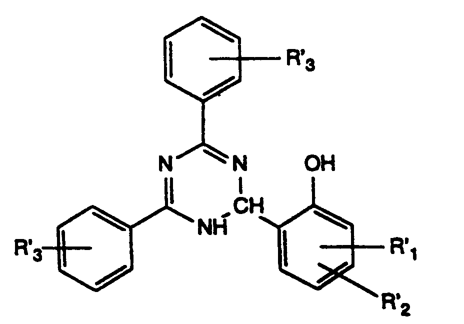 dans laquelle
R'₁, R'₂ et R'₃ représentent, indépendamment les uns des autres, des atomes d'hydrogène, des atomes d'halogène, des groupes alkyle en C₁-C₁₈, alkoxy en C₁-C₁₈ ou C≡N, à condition que R'₁, R'₂ et R'₃ ne représentent pas des atomes d'hydrogène simulatnément, en tant que produit intermédiaire pour la préparation d'absorbeurs UV de formule (1) selon la revendication 1.
